# NOUVEAU FASCICULE DE BREVET EUROPEEN

(11) **EP 0 459 900 B2**
(45) Date de publication et mention de la décision concernant l'opposition: **28.08.2002**
(45) Mention de la délivrance du brevet: 05.01.1994
(21) Numéro de dépôt: 91401397.4
(22) Date de dépôt: 30.05.1991
(51) Int. Cl.: A61K 7/13

(54) **Procédé de teinture des fibres kératiniques avec 1e 2,4-diamino 1,3-diméthoxybenzène à pH acide et compositions mises en oeuvre**
Verfahren zum Färben von Keratinfasern mit 2,4-Diamino-1,3-dimethoxybenzol in einem sauren Medium sowie Mittel zur Durchführung des Verfahrens
Process for dyeing of keratinic fibres with 2,4-diamino-1,3-dimethoxybenzene in an acid medium and composition using this process

(30) Priorité: 31.05.1990 FR 9006801
(43) Date de publication de la demande: 04.12.1991
(73) Titulaire: L'OREAL, 75008 Paris (FR)
(72) Inventeur: Cotteret, Jean, F-78480 Verneuil-sur-Seine (FR); Audousset, Marie Pascale, F-92300 Levallois-Perret (FR)
(74) Mandataire: Casalonga, Axel

(56) Documents cités:
- EP-A- 0 370 880
- WO-A-87/01033
- WO-A-88/01161
- WO-A-88/01162
- FR-A- 2 615 730
- FR-A- 2 615 732

## Description

La présente invention est relative à un procédé de teinture des fibres kératiniques, en particulier des fibres kératiniques humaines telles que les cheveux, mettant en oeuvre le 2,4-diamino 1,3-diméthoxybenzène en association avec des bases d'oxydation et un agent oxydant en milieu acide et aux compositions mises en oeuvre au cours de ce procédé.

Il est connu de teindre les fibres kératiniques et en particulier les cheveux humains avec des compositions tinctoriales contenant en milieu alcalin, des précurseurs de colorants d'oxydation et en particulier des p-phénylènediamines, des ortho ou para-aminophénols appelés généralement "bases d'oxydation".

On sait également que l'on peut faire varier les nuances obtenues avec ces bases d'oxydation en les associant à des coupleurs encore appelés modificateurs de coloration, choisis notamment parmi les métadiamines aromatiques, les méta-aminophénols et les méta-diphénols.

La demanderesse a déjà proposé pour la teinture, en milleu oxydant alcalin, comme coupleur le 2,4-diamino 1,3-diméthoxybenzène (FR-A-2 615 732).

La demanderesse vient de découvrir que l'utilisation de ce coupleur avec des bases d'oxydation en un mélange extemporané avec un oxydant, à un pH acide, permettait d'obtenir une puissance tinctoriale au moins égale à celle obtenue antérieurement à pH alcalin et une remarquable stabilité des teintures à la lumière, aux lavages, à la transpiration et aux intempéries.

La présente invention a donc pour objet un procédé de teinture des fibres kératiniques, en particulier des fibres kératiniques humaines telles que les cheveux comprenant l'application sur ces fibres d'au moins une composition contenant le 2,4-diamino 1,3-diméthoxybenzène, un précurseur de colorant d'oxydation, à pH acide.

L'invention a également pour objet un agent de teinture à deux composants, dont l'un des composants comprend le 2,4-diamino 1,3-diméthoxybenzène et le précurseur de colorant d'oxydation et l'autre l'agent oxydant à un pH acide, et en des quantité telle que le mélange présents un pH acide.

L'invention a également pour objet la composition prévue à l'emploi, contenant les différents agents utilisés pour la teinture, en milieu acide, des cheveux.

D'autre objet de l'invention apparaîtront à la lecture de la description et des exemples qui suivent.

Le procédé de teinture des fibres kératiniques et en particulier des fibres kératiniques humaines telle que les cheveux, conforme à l'invention, est essentiellement caractérisé par le fait que l'on applique sur ces fibres une composition constituée par de l'eau ou un mélange d'eau et d'un solvant choisi parmi les alcanols inférieurs en C2-C4, le glycérol, les glycols ou éther de glycols, le monoéthyléther et le monoéthyléther du diéthylèneglycol, les alcools aromatiques ou leurs mélanges et éventuellement des agents tensioactifs anioniques, cationiques, non ioniques, amphotères ou leurs mélanges, des épaississants, des agents antioxydants, des agents de pénétration, des agents séquestrants, des conservateurs et des parfums,
- au moins, comme coupleur, le 2,4-diamino 1,3-diméthoxybenzène ou l'un de ses sels ;
- un ou plusieurs précurseur de colorant d'oxydation ;
- un agent oxydant constitué par le peroxyde d'hydrogène ;
- le pH de la composition appliquée sur les fibres étant inférieur à 7."

La composition ne contient pas d'ions iodure en quantité suffisante pour oxyder le 2,4-diamino 1,3-diméthoxybenzène.

Les sels sont choisis parmi les sels d'addition d'acides, tels que l'acide chlorhydrique, sulfurique, etc.,

Les précurseurs de colorants d'oxydation ou bases d'oxydation sont des comprosés connus qui ne sont pas des colorants en eux-même et qui forment un colorant par un processus de condensation oxydative, soit sur eux-même, soit en présence d'un coupleur ou modificateur. Ces composés comportent généralement un noyau aromatique portant des groupement fonctionnels, constitués : solt par deux groupements amino; soit par un groupement amino et un groupement hydroxy, ces groupement étant en position para ou ortho, l'un par rapport à l'autre.

Les précurseurs de colorants d'oxydation de type para, utilisés conformément à l'invention, sont choisis parmi les paraphénylènediamines, les paraaminophénols, les précurseurs hétérocycles para, comme la 2,5-diaminopyridine, la 2-hydroxy 5-amino pyridine, la 2,4,5,6-tétraaminopyrimidine.

Parml les paraphénylénediamines, on peut citer les composés répondant à la formule (1) : 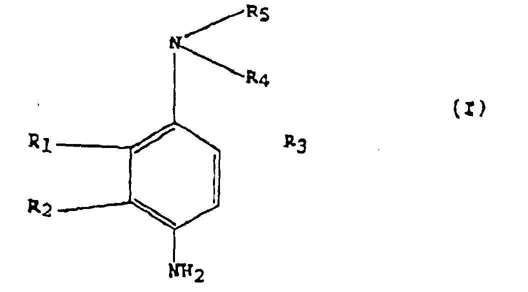 dans laquelle R₁, R₂ et R₃, Identiques ou différents, représentant un atome d'hydrogène ou d'halogène, un radical alkyle ayant de 1 à 4 atomes de carbone, un radical alcoxy ayant de 1 à 4 atomes de carbone; R₄ et R₅, Identiques ou différents. représentent un atome d'hydrogène, un radical alkyle, hydroxyalkyle, alcoxy alkyle, carbamylalkyle, mésylaminoalkyle, acétylamino alkyle, uréidoalkyle, carbalcoxyaminoalkyle, pipéridino alkyle, morpholinoalkyle, ces groupes alkyle ou alcoxy ayant de 1 à 4 atomes de carbone, ou bien R₄ et R₈ forment. conjointement avec l'atome d'azote auquel ils sont liés, un hétérocycle pipéridino ou morpholino, sous réserve que R₁ ou R₃ représente un atome d'hydrogène lorsque R₄ et R₆ ne représentent pas un atome d'hydrogène, ainsi que les sels de ces composés à l'exclusion de la 2,6-diméthylparaphénylènediamine et de la 2,3-diméthylparaphénylènediamine.

Parmi les composés préférés répondant à la formule (1), on peut citer l'isopropyl-p-phénylène diamine, la p-phénylènediamine, la 2-méthyl-p-phénylène diamine, la méthoxyparaphénylènediamine, la chloroparaphénylènediamine, la 2-méthyl 5-méthoxy-paraphénylènediamine, la 2,6-diméthyl 5-méthoxyparaphénylènediamine, la N,N-diméthylparaphénylènediamine, la 3-méthyl 4-amino N,N-diéthylaniline, la N,N-di-(β-hydroxyéthyl)paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl, carbamylméthyl)aniline, la 3-méthyl 4-amino N,N-(éthyl,carbamylméthyl)aniline, la 4-amino N,N-(éthyl,β-pipéridinoéthyl)aniline, la 3-méthyl 4-amino N,N-(éthyl,β-pipéridinoéthyl)aniline, la 4-amino N,N-(éthyl,β-morpholinoéthyl)anillne, la 3-méthyl 4-amino N,N-(éthyl,β-morphclinoéthyl)aniline, la 4-amino N,N-(éthyl,β-acétylaminoéthyl)aniline la 4-amino N-(β-méthoxyéthyl)anilin, la 3-méthyl 4-amino N,N-(éthyl,β-acétylaminoéthyl)anillne, la 4-amino N,N(éthyl,β-mésylaminoéthyl)aniline, la 3-méthyl 4-amino N,N-(éthyl,β-mésylaminoéthyl)aniline, la 4-amino N,N-(éthyl,β-sulfoéthyl)aniline, la 3 méthyl 4-amino N,N-(éthyl,β-sulfoéthyl)aniline, la N- (4-amino phényl) morpholine, la N- (4-amino phényl)pipéridine.

Ces précurseurs de colorants par oxydation de type para peuvent être Introduits dans la composition tinctoriale, soit sous forme de base libre, soit sous forme de sels, tels que sous forme de chlorhydrate, de bromhydrate ou de sulfate.

Parmi les p-aminophénols, on peut citer le p-aminophénol, le 2-méthyl 4-aminophénol, le 3-méthyl 4-aminophénol, le 2-chloro 4-aminophénol, le 3-chloro 4-aminophénol, le 2,6-diméthyl 4-aminophénol, le 3,5-diméthyl 4-aminophénol, le 2,3-diméthyl 4-aminophénol, le 2-hydroxyméthyl 4-aminophénol, le 2-(β-hydroxyéthyl) 4-aminophénol, le 2-méthoxy 4-aminophénol, le 3-méthoxy 4-aminophénol, le 2,5-diméthyl 4-aminophénol, le 2-méthoxyméthyl 4-aminophénol.

Les précurseurs de colorants d'oxydation de type ortho sont choisis parmi les orthoaminophénols, comme le 1-amino 2-hydroxybenzène, le 6-méthyl 1-hydroxy 2-aminobenzène, le 4-méthyl 1-amino 2-hydroxybenzène et les orthophénylènediamines.

Le pH de la composition appliquée sur les fibres kératiniques, en particulier les cheveux, à une valeur inférieure à 7 et est compris de préférence entre 3 et 6.9. Ce pH est ajusté par l'utilisation d'agents acidifiants bien connus dans le domaine de la teinture des fibres kératiniques, et en particulier des cheveux humains, tels que des acides minéraux ou organiques comme l'acide chloridrique, l'acide phosphorique, les acides carboxyliques comme l'acide tartrique, l'acide citrique ou les acides sulfoniques.

Le 2,4-diamino 1,3-diméthoxybenzène est présent dans la composition appliquée sur les fibres kératiniques, dans des proportions comprises de préférence entre 0,01 et 3,5% en poids par rapport au poids total de la composition.

Les compositions définies ci-dessus appliquées dans la teinture des fibres kératiniques, peuvent également contenir en plus du 2,4 diamino 1,3-diméthoxy-benzène, d'autres coupleurs connus en eux-mêmes tels que des métadiphénols, des métaaminophénols, des métaphénylènediamines, des méta N-acylaminophénols, des métauréldophénols, des métacarbalcoxyaminophénols, l'α-naphtol, des coupleurs possédant un groupement méthylène actif, tels que lee composés dicétoniques, les pyrazolones.

Parmi ces coupleurs pouvant être utilisés en plus du 2,4-diamino 1,3-diméthoxybenzène, on peut citer le 2,4-dihydroxyphénoxyéthanol, le 2,4-dihydroxy anisole, le métaaminophénol, la résorcine, le monométhyléther de résorcine, la 2-méthylrésorcine, le pyrocatéchol, le 2-méthyl 5-N-(β-hydroxyéthyl)amino phénol, le 2-méthyl 5-N-(β-mésylaminoéthyl)aminophénol, la 6-hydroxybenzomorpholine, le 2,4-diaminoanisole, le 2,4-diaminophénoxyéthanol, la 6-aminobenzomorpholine, le [2-N-(β-hydroxyéthyl)amino 4-amino]-phénoxyéthanol, le 2-amino 4-N-(β-hydroxyéthyl)aminoanisole, le (2,4-diamino)phényl-β, α-dihydroxypropyléther, la 2.4-diaminophénoxyéthylamine, le 2-méthyl 5-aminophénol, le 2,8-diméthyl 3-aminophénol, le 3,4-méthylènedioxyphénol, la 3,4-méthylènedioxyaniline et leurs sels.

Ces compositions peuvent également contenir des agent tensio-actifs anioniques, cationiques, non ioniques, amphotères ou leurs mélanges.

Parmi ces agents tensio-actifs, on peut citer les alkylbenzènesulfonates, les alkylnaphtalène sulfonates, les sulfates, les éthersulfates et les aulfonates d'acools gras, les sels d'ammonium quatemaires tels que le bromure de triméthylcéthyl ammonium, le bromure de cétylpyridinium, les éthanolamides d'acides gras éventuellement oxyéthylénés, les acides, les alcools ou les amines polyoxyéthylénés, les alcools polyglycérolés, les alkylphénols polyoxyéthylénés ou polyglycérolés, ainsi que les alkylsulfates polyoxyéthylénés.

Les compositions tinctoriales sont généralamant aqueuses, mais elles peuvent également contenir des solvants organiques pour solubiliser des composés qui ne seraient pas suffisamment solubles dans l'eau. Parmi ces solvant, on peut citer à titre d'exemple, les alcanols Inférieurs en C₂-C₄ tels que l'éthanol et l'Isopropanol, le glycérol, les glycols ou éthers de glycols. comme le butoxy-2 éthanol, l'éthylène glycol, le propylèneglycol, le monoéthyléther et le monométhyléther du diéthylèneglycol, ainsi que les alcools aromatiques comme l'alcool benzylique ou le phénoxyéthanol, ou les mélanges de ces solvants.

La composition appliquée sur les cheveux peut également renfermer des agents épaississants choisis en particulier parmi l'alginate de sodium, la gomme arabique, les dérivés de cellulose tels que la méthylcellulose, l'hydroxyéthylcellulose, l'hydroxypropylcellulose, l'hydroxyméthylcellulose, la carboxyméthylcellulose, les polymères d'acide acrylique éventuellement réticulés, la gomme de xanthane. On peut également utiliser des agents épaississants minéraux tels que la bentonite.

La composition peut également renfermer les agent antioxydants choisis en particulier parmi le sulfite de sodium, l'acide thioglycolique, le bisulfite de sodium, l'acide ascorbique et l'hydroquinone, ainsi que d'autres adjuvants cosmétiquement acceptables lorsque la composition est destinée a être utillsée pour la teinture des fibres kératiniques humaines, tels que des agents de pénétration, des agents séquestrants, des conservateurs, des tampons, des parfums, etc...

La composition appliquée sur les cheveux peut se présenter sous des formes diverses, telles que de liquides, de crèmes, de gels ou sous toute autre forme appropriée pour réaliser une teinture capillaire. Elle peut être conditionnée en flacon aérosol en présence d'un agent propulseur.

L'Invention a également pour objet la composition prête à l'emploi utilisée dans le procédé défini ci-dessus.

Selon une forme de réalisation préférée, le procédé comporte une étape préliminaire consistant à stocker sous forme séparée, d'une part, la composition constituée par de l'eau ou un mélange d'eau et d'un solvant choisi parmi les alcanols inférieurs en C2-C4, le glycérol, les glycols ou éther de glycols, le monoéthyléther et le monoéthyléther du diéthylèneglycol, les alcools aromatiques ou leurs mélanges et éventuellement des agents tensioactifs anioniques, cationiques, non ioniques, amphotères ou leurs mélanges, des épaississants, des agents antioxydants, des agents de pénétration, des agents séquestrants, des conservateurs et des parfums, le coupleur 2,4-diamino 1,3-diméthoxybenzene et les précurseurs de colorants d'oxydation sous forme d'un composant (A) et, d'autre part, une composition renfermant l'agent oxydant tel que défini ci-dessus sous forme d'un composant (B), et à procéder à leur mélange extemporané avant d'appliquer ce mélange sur les fibres kératiniques, comme indiqué ci-dessus. Le composant (A) ne contient pas d'ion iodure en quantité suffisante pour oxyder le 2,4-diamino 1,3-diméthoxybenzène.

La composition appliquée sur les fibres kératiniques résulte d'un mélange da 10 à 90% du composant (A) avec 90 à 10% du composant (B) contenant un agent oxydant

L'invention a également pour objet un agent de teinture des fibres kératiniques, en particulier des cheveux, essentiellement caractérisé par le fait qu'il est constitué de deux composants, l'un des composants étant constitué par le composant(A) défini ci-dessus et l'autre étant constitué par le composant (B) également défini ci-dessus, la pH des composants (A) et (B) étant tel qu'après mélange dans des proportions de 90 à 10% pour le composant (A) et de 10 à 90% pour le composant (B), la composition résultante ait un pH inférieur à 7.

Dans cette forme de réalisation, le composant (A) qui renferme au moins le 2,4-diamino 1,3-diméthoxybenzène et un précurseur de colorant d'oxydation, a un pH compris entre 3 et 10,5 et peut être ajusté à la valeur choisie au mayen d'agents alcalinisants habituellement utillsés en teinture des fibres kératiniques, tels que l'ammoniaque, les carbonates alcalins, les alcanolamines tels que les mono-, di- et triéthanolamines ainsi que leurs dérivés au des agents acidifiants classiques, tels que les acides minéraux ou organiques, comme les acides chlorhydrique, phosphorique, les acides carboxyliques tels que l'acide tartrique ou citrique ou les acides sulfoniques.

Cette composition peut renfermer les différents autres adjuvants mentionnés ci-dessus, notamment des coupleurs différents du coupleur 2,4-diamino 1,3-diméthoxybenzène.

L'ensemble des précurseurs de colorants par oxydation du type para et/ou ortho ainsi que les coupleurs, sont présents dans des proportions comprises de préférence entre 0,3 et 7% en poids par rapport au poids total du composant (A). La concentration en 2,4-diamino 1,3-diméthoxybenzène peut varier entre 0,05 et 3,5% en poids par rapport au poids total du composant (A).

Les agents tensio-actifs sont présents dans le composant (A) dans des proportions de 0,1 à 55% en poids. Lorsque le milleu contient des solvants en plus de l'eau, ceux-ci sont présents dans des proportions comprises entre 0,5 et 40% en poids et en particulier entre 5 et 30% en poids par rapport au poids total du composant (A). Les agents épaississants sont présents do préférence dans des proportions comprises entre 0,1 et 5%, et en particulier entre 0,2 et 3% en poids. Les agents anti-oxydants mentionnés ci-dessus sont de préférence présents dans le composant (A) dans des proportions comprises entre 0,02 et 1,5% en poids par rapport au poids total du composant (A).

Les composants (B) renfermant l'agent oxydant tel que défini ci-dessus, a un pH inférieur à 7. Co pH peut avoir une valeur minimum de 1 et de préférence il est compris entre 1,5 et 3,5. Ce composant (B) peut être acidifié avec la même type d'agents acidifiants que ceux utilisés pour le composant (A).

Il peut se présenter sous forme de liquide plus ou moins épaissi, de lait ou de gel.

Cet agent de teinture à deux composants peut être conditionné dans un dispositif à plusieurs compartiments ou kit de teinture, ou tout autre système de conditionnement à plusieurs compartiments dont l'un renferme la composant (A) et le second renferme le composant (B); ces dispositifs pouvant être équipés d'un moyen permettant de déilvrer sur les cheveux le mélange souhaité, tel que les dispositifs décrits dans le brevet US-A-4 823 985 de la demanderesse.

Conformément à l'invention, le procédé de teinture consiste à appliquer sur les chevaux le mélange obtenu, à le laisser poser pendant 3 à 40 minutes, puis à rincer les cheveux et éventuellement effectuer un shampoing.

Il est également possible, conformément à l'invention, d'appliquer séparément une composition contenant le coupleur 2,4-diamino 1,3-diméthoxybenzène, le précurseur de colorant d'oxydation et l'agent oxydant, de façon à ce que le mélange se formant in-situ au niveau des fibres ait un pH inférieur à 7, comme défini ci-dessus.

Les exemples suivants sont destinés à illustrer l'invention sans pour autant présenter un caractère limitatif.

### EXEMPLES 1 à 6

On procède à la teinture de, cheveux en appliquant sur des cheveux permanentés gris à 90% de blancs, un mélange axtemporané de la composition colorante (A) et de la composition oxydante (B).

Ce mélange présente le pH indiqué dans le tableau des exemples qui suivent

On laisse agir ce mélange pendant 30 minutes, puis on rince les cheveux, on effectue un shampooing.

Après séchage, les cheveux sont teints dans la nuance précisée au bas du tableau.

| en g | 1 | 2 | 3 | 4 | 5 |
|---|---|---|---|---|---|
| A) Composition colorante | | | | | |
| 2,4-diamino 1,3-diméthoxybenzène, 2 HCl | 0,723 | 0,482 | 0,241 | 0,723 | 0,723 |
| Paraphénylènediamine | 0,324 | | | 0,.324 | |
| Paraaminophénol | | 0,436 | | | |
| 2-méthylparaphénylènediamine, 2 HCl | | | 0,488 | | 0,555 |
| 2-méthyl 5-N-(β-hydroxyéthylamino)phénol | | 0,334 | | | |
| Métaaminophénol | | | 0,144 | | |
| a-naphtol | | | 0,109 | | |
| Monoéthanolamine qs pH | 9,8 | 9,7 | 9,1 | 9,0 | 8,7 |
| Support 1 | X | X | | | |
| Support 2 | | | X | X | X |
| Eau gsp | 100 | 100 | 100 | 100 | 100 |

| | 1 | 2 | 3 | 4 | 5 |
|---|---|---|---|---|---|
| B) Composition oxydante | | | | | |
| Solution d'eau oxygénée a 20 volumes | | | | | |
| Acide phesphorique qs pH | 1.1 | 1,4 | 1,2 | 1,2 | 1,2 |
| pH mélange P/P A + 3 | 5,5 | | 6,5 | 6,4 | 6 |
| pH mélange 1/3 A + 2/3 3 | | 4,8 | | | |
| Nuances obtenues : | bleu nuit puissant: | rose | bleu puissant | bleu puissant | bleu puissant |

### EXEMPLE 6

- 2,4-diamino 1,3-diméthoxybenzène 0,1 g
- Paraphénylènediamine 0,4 g
- Métaaminophénol 0,3 g
- Alcool cétylstéarylique 18,0 g
- 2-octyldodécanol 3,0 g
- Alcool cétylstéarylique oxyéthyléné à 15 moles d'oxyde d'éthylène 3,0 g
- Laurylsulfate d'ammonium 3,6 g
- Monoéthanolamine 3,0 g
- Séquestrant antioxydant qs
- Eau qsp 100,0 g

La pH de cette composition est égal à 10,2.

Au moment de l'emploi on mélange poids par poids, la composition ci-dessus, avec une solution d'eau oxygénée à 20 volumes dont le pH est ajusté entre 1 et 1,5 (par addition de 2.5 g d'acide phosphorique à 100 g de solution d'eau oxygénés).

Le pH de la composition après mélange est égal à 8,8. Ce mélange est appliqué sur des cheveux gris à 90% de blancs pendant 30 minutes.

Après rinçage, lavage et séchage, les cheveux sont colorés en châtain cendré.

### SUPPORT DE COLORATION 1

- Nonylphénol à 4 moles d'oxyde d'éthylène, vendu sous la dénomination "SINNOPAL NP4" par la Société HENKEL 25,5 5 g
- Nonylphénol à 9 moles d'oxyde d'éthylène, vendu sous la dénomination "SINNOPAL NP9" par la Société HENKEL 17,5 g
- Monoéthyléther d'éthylèneglycol 7,0 g
- Propylèneglycol 10,5 g
- Dipropylèneglycol 0,5 g
- Alcool éthylique 2,0 g
- Lauryl éther sulfate de monoéthenolamine, vendu sous la dénomination "SACTIPON 2 OM 29" par la Société LEVER à 28% de MA 4,2 g MA
- ALkyl éther sulfate de sodium à 28% de MA 0,8 g MA
- Métabisulfite de sodium en solution aqueuse à 35% de MA 0,45 g MA
- Acétate de sodium 0,8 g
- Antioxydant, séquestrant qs

### SUPPORT DE COLORATION 2

- Alcool oléique polyglycérolé à 2 moles de glycérol 4,0 g
- Alcool oléique polyglycérolé à 4 moles de glycérol à 78% de MA 5,69 g MA
- Acide oléique 3,0 g
- Amine oléique à 2 moles d'oxyde d'éthylène, vendue sous la dénomination "ETHOMEEN 0 12" par la Société AKZO 7.0 g
- Laurylamino succinamate de diéthylaminopropyle, sel de sodium à 55% de MA 3,0 g MA
- Alcool oléique 5,0 g
- Diéthanolamide d'acide oléique 12,0 g
- Propylèneglycol 3,5 g
- Alcool éthylique 7,0 g
- Dipropylèneglycol 0,5 g
- Monométhyléther de propylène glycol 9,0 g
- Métabisulfite de sodium en solution aqueuse à 35% de MA 0,45 g MA
- Acétate d'ammonium 0,8 g
- Antioxydant, séquestrant qs

## Revendications

1. Procédé de teinture des fibres kératiniques, en particulier des fibres kératiniques humaines telles que les cheveux, **caractérisé par le fait qu'**on applique sur ces fibres une composition constitué par de l'eau ou un mélange d'eau et d'un solvant choisi parmi les alcanols inférieurs en C₂-C₄, le glycérol, les glycols ou éther de glycols, le monoéthyléther et le monométhyléther du diéthylèneglycol, les alcools aromatiques ou leurs mélanges et éventuellement des agents tensioactifs anioniques, cationiques, non ioniques, amphotères ou leurs mélanges, des épaississants, des agents antioxydants, des agents de pénétration, des agents séquestrants, des conservateurs et des parfums,
- au moins, comme coupleur, le 2,4-diamino 1,3-diméthoxybenzène ou l'un de ses sels ;
- un ou plusieurs précurseurs de colorant d'oxydation ;
- un agent oxydant constitué par le peroxyde d'hydrogène;
- le pH de la composition appliquée sur les fibres étant inférieur à 7.

2. Procédé selon la revendication 1, **caractérisé par le fait que** les précurseurs de colorants d'oxydation sont choisis parmi les paraphénylène diamines. les para-aminophénols, les précurseurs hétérocycliques para.

3. Procédé selon la revendication 2, **caractérisé par le fait que** les paraphénylènedlamines sont choisies parmi les composés répondant à la formule: 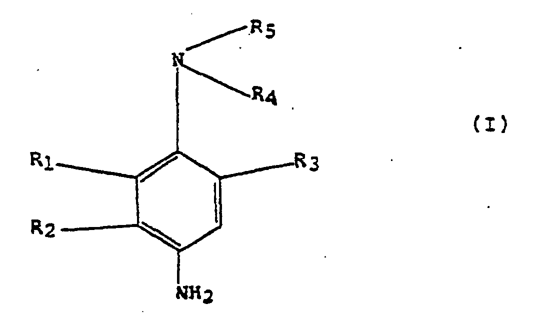 dans laquelle R₁, R₂ et R₃, identiques ou différents, représentent un atome d'hydrogène ou d'halogène, un radical alkyle ayant de 1 à 4 atomes de carbone, un radical alcoxy ayant de 1 à 4 atomes de carbone; R₄ et R₅, identiques ou différents; représentent un atome d'hydrogène, un radical alkyle, hydroxyalkyle, alcoxy alkyle, carbamylalkyle, mésylaminoalkyle, acétylamino alkyle, uréidoalkyle, carbalcoxyaminoalkyle, pipéridino alkyle, morpholinoalkyle, ces groupes alkyle ou alcoxy ayant de 1 à 4 atomes de carbone, ou bien R₄ et R₅ forment, conjointement avec l'atome d'azote auquel ils sont liés. un hétérocycle pipéridino ou morpholino, sous réserve que R₁ ou R₃ représente un atome d'hydrogène lorsque R₄ et R₅ ne représentent pas un atome d'hydrogène, ainsi que les sels de ces composés, à l'exclusion de la 2,6-diméthylparaphénylènediamine et de la 2,3-diméthylparaphénylènediamine.

4. Procédé selon la revendication 2 ou 3, **caractérisé par le fait que** les composés de formule (I) sont choisis parmi l'isopropyl-p-phénylènediamine, la p-phénylènediamine, la 2-méthyl-p-phénylènediamine, la méthoxyparaphénylènediamine, la chloroparaphénylène diamine, la 2-méthyl 5-méthoxyparaphénylènediamine, la 2,6-diméthyl 5-méthoxyparaphénylènediamine, la N,N-diméthylparaphénylènediamine, la 3-méthyl 4-amino N,N-diéthylaniline, la N,N-di-(β-hydroxyéthyl)paraphénylènediamine, la 3-méthyl 4-amino N,N-di-(β-hydroxyéthyl)aniline, la 3-chloro 4-amino N,N-di-(β-hydroxyéthyl)aniline, la 4-amino N,N-(éthyl,carbamylméthyl) aniline, la 3-méthyl 4-amino N,N-(éthyl,carbamylméthyl)aniline, la 4-amino N,N-(éthyl,β-pipéridinoéthyl)aniline, la 3-méthyl 4-amino N,N-(éthyl,β-pipéridinoéthyl)aniline, la 4-amino N,N-(éthyl,β-morpholinoéthyl)aniline, la 3-méthyl 4-amino N,N-(éthyl,β-morpholinoéthyl)aniline, la 4-amino N,N-(éthyl,β-acétylaminoéthyl)aniline, la 4-amino N-(β-méthoxyéthyl)aniline, la 3-méthyl 4-amino N,N-(éthyl,β-acétylaminoéthyl)aniline, la 4-amino N,N-(éthyl,β-mésylaminoéthyl)aniline, la 3-méthyl 4-amino N,N-(éthyl,β-mésylaminoéthyl)aniline, la 4-amino N,N-(éthyl,β-sulfoéthyl)aniline, la 3-méthyl 4-amino N,N-(éthyl,β-sulfoéthyl)aniline, la N- (4 -amino phényl)morpholine, la N- (4 -amino phényl) pipéridine, sous forme de base libre ou de sels.

5. Procédé selon la revendication 2 ou 3, **caractérisé par le fait que** les p-aminophénols sont choisis parmi le p-aminophénol, le 2-méthyl 4-amino phénol, le 3-méthyl 4-aminophénol, le 2-chloro 4-amino phénol, le 3-chloro 4-aminophénol, le 2,6-diméthyl 4-aminophénol, le 3,5-diméthyl 4-aminophénol, le 2,3-diméthyl 4-aminophénol, le 2-hydroxyméthyl 4-aminophénol, le 2-(β-hydroxyéthyl)4-aminophénol, le 2-méthoxy 4-aminophénol, le 3-méthoxy 4-aminophénol, le 2,5-diméthyl 4-aminophénol, le 2-méthoxyméthyl 4-aminophénol.

6. Procédé selon la revendication 1, **caractérisé par le fait que** les précurseurs de colorants d'oxydation sont des précurseurs de colorants d'oxydation de type ortho choisis parmi les ortho-aminophénols et les orthophénylènediamines.

7. Procédé selon l'une quelconque des revendications 1 à 6, **caractérisé par le fait que** le pH de la composition appliquée sur les fibres kératiniques est compris entre 3 et 6,9.

8. Procédé selon l'une quelconque des revendications 1 à 7, **caractérisé par le fait que** la composition utilisée pour la teinture des fibres kératiniques contient en plus du coupleur 2,4-diamino 1,3-diméthoxybenzène, d'autres coupleurs choisis parmi les métadiphénols, les métaaminophénois, les métaphénylèdiamines, les méta N-acylaminophénols, les métauréidophénois, les métacarbaicoxyaminophénois, l'α-naphtol, les coupleurs possédant un groupement méthylène actif choisis parmi les composés dicétoniques et les pyrazolones.

9. Procédé selon la revendication 8, **caractérisé par le fait que** les coupleurs sont choisis parmi le 2,4 dihydroxyphénoxyéthanol, le 2,4-dihydroxyanisole, le métaaminophénol, la résorcine, le monométhyléther de résorcine, la 2-méthylrésorcine, le pyrocatéchol, le 2-méthyl 5-N-(β-hydroxyéthyl)amino phénol, le 2-méthyl 5-N-(β-mésylaminoéthyl)aminophénol, la 6-hydroxybenzomorpholine, le 2,4-diaminoanisole, le 2,4-diaminophénoxyéthanol, la 8-aminobenzomorpholine, le [2-N-(β-hydroxyéthyl)amino 4-amino]-phénoxyéthanol, le 2-amino 4-N-(β-hydroxyéthyl)aminoanisole le (2,4-diamino)phényl-β, α-dihydroxypropyléther, la 2,4-diaminophénoxyéthylamine, le 2-méthyl 5-aminophénol, le 2,6-diméthyl 3-aminophénol, le 3,4-méthylènedioxyphénol, la 3,4-méthylènedioxyaniline et leurs sels.

10. Procédé selon l'une quelconque des revendications 1 à 9, **caractérisé par le fait que** la composition contient des agents tensio-actifs anioniques, cationiques, non ioniques, amphotères ou leurs mélanges; des agents épaississants, des agents anti-oxydants et/ou tout autre adjuvant cosmétiquement acceptable.

11. Agent de teinture des fibres kératiniques et en particulier des cheveux, **caractérisé par le fait qu'**il est constitué par deux composants : un composant (A) constitué par une composition constitué par de l'eau ou un mélange d'eau et d'un solvant choisi parmi les alcanols inférieurs en C₂-C₄, le glycérol, les glycols ou éther de glycols, le monoéthyléther et le monométhyléther du diéthylèneglycol, les alcools aromatiques ou leurs mélanges et éventuellement des agents tensioactifs anioniques, cationiques, non ioniques, amphotères ou leurs mélanges, des épaississants, des agents antioxydants, des agents de pénétration, des agents séquestrants, des conservateurs et des parfums - au moins comme coupleur le 2,4-diamino 1,3-diméthoybenzène et un précurseur de colorant d'oxydation tel que défini dans l'une quelconque des revendications 2 à 6, un composant (B) constitué par un agent oxydant constitué par le peroxyde d'hydrogène, le pH des composants (A) et (B) étant tel qu'après mélange dans des proportions de 90 à 10% pour le composant (A), de 10 à 90% pour le composant (B), la composition résultante ait un pH inférieur à 7.

12. Agent selon la revendication 11, **caractérisé par le fait que** le composant (A) a un pH compris entre 3 et 10,5.

13. Agent selon la revendication 11 ou 12, **caractérisé par le fait que** le composant (A) contient les précurseurs de colorants par oxydation du type para et/ou ortho ainsi que les coupleurs, dans des proportions comprises entre 0,3 et 7% en poids par rapport au poids total du composant (A).

14. Agent selon l'une quelconque des revendications 11 à 13, **caractérisé par le fait que** la concentration en 2,4-diamino 1,3-diméthoxybenzène est comprise entre 0,05 et 3,5% en poids par rapport au poids total du composant (A).

15. Agent selon l'une quelconque des revendications 11 à 14, **caractérisé par le fait que** le composant (A) contient des agents tensio-actifs dans des proportions de 0,1 à 55% en poids, des agents solvants en plus de l'eau dans des proportions comprises entre 0,5 et 40% en poids, des agents épaississants dans des proportions comprises entre 0,1 et 5% en poids, des agents antioxydants dans des proportions comprises entre 0,02 et 1,5% en poids, et/ou tout autre adjuvant cosmétiquement acceptable.

16. Agent selon l'une quelconque des revendications 11 à 15, **caractérisé par le fait que** le composant (B) a un pH qui a une valeur minimum de 1 et inférieure à 7.

17. procédé de teinture des fibres kératiniques et en particulier des cheveux, **caractérisé par le fait qu'**il consiste en une première étape consistant à stocker sous forme séparée les composants de l'agent de teinture tel que défini dans l'une quelconque des revendications 11 à 16 et a procéder avant application au mélange des composants (A) et (B) dans des proportions de 10 à 90% pour le composant (A) et de 90 à 10% pour le composant (B), de façon à obtenir une composition ayant un pH inférieur à 7 et d'appliquer ce mélange immédiatement après préparation sur les fibres kératiniques.

18. Dispositif à plusieurs compartiments ou kit de teinture, **caractérisé par le fait qu'**il consiste en deux compartiments dont un premier compartiment renferme le composant (A) tel que défini dans l'une quelconque des revendications 11 à 16 et le second compartiment renferme le composant (B) tel que défini dans les revendications 11 à 17.

19. Dispositif selon la revendication 18, **caractérisé par le fait qu'**il est équipé d'un moyen parmettant de délivrer sur les cheveux le mélange souhaité des composant (A) et (B).

20. Procédé de teinture selon l'une quelconque des revendications 1 à 10, **caractérisé par le fait que** l'on applique sur les cheveux la composition et qu'on la laisse poser pendant 3 à 40 minutes, que l'on rince les cheveux et qu'on procède éventuellement à un shampooing avant un nouveau rinçage et séchage.

21. 24. Composition de teinture de fibres kératiniques, prête à l'emploi telle que mise en oeuvre dans la procédé selon l'une quelconque des revendications 1 à 10.

22. Composition selon la revendication 21, contenant le 2,4-diamino 1,3-diméthoxybenzène dans des proportions de 0,01 à 3,5% en poids par rapport au poids total de la composition.

## Patentansprüche

1. Verfahren zur Färbung keratinischer Fasern, insbesondere menschlicher keratinischer Fasern wie der Haare, **dadurch gekennzeichnet, dass** man auf diese Fasern eine Zusammensetzung, die aus Wasser oder einer Mischung aus Wasser und einem Lösungsmittel, ausgewählt aus C₂₋₄-Niedrigalkanolen, Glycerin, Glycolen oder Glycolethern, Diethylenglycolmonoethylether und -monomethylether, aromatischen Alkoholen oder aus deren Mischungen, und gegebenenfalls aus anionischen, kationischen, nicht-ionischen, amphoteren oberflächenaktiven Mitteln oder deren Mischungen, Verdickungsmitteln, Antioxidantien, Eindringmitteln, Sequestriermitteln, Konservierungsstoffen und aus Parfümen zusammengesetzt ist,
- mindestens 2,4-Diamino-1,3-dimethoxybenzol oder eines seiner Salze als Kuppler,
- mindestens eine oder mehrere Oxidationsfarbstoff-Vorstufenverbindungen und
- ein oxidierendes Mittel, das aus Wasserstoffperoxid zusammengesetzt ist, aufbringt, wobei
- der pH-Wert der auf die Fasern aufgetragenen Zusammensetzung weniger als 7 beträgt.

2. Verfahren gemäß Anspruch 1, **dadurch gekennzeichnet, dass** die Oxidationsfarbstoff-Vorstufenverbindungen aus p-Phenylendiaminen, p-Aminophenolen und aus heterozyklischen p-Vorstufenverbindungen ausgewählt sind.

3. Verfahren gemäß Anspruch 2, **dadurch gekennzeichnet, dass** die p-Phenylendiamine ausgewählt sind aus Verbindungen der Formel: 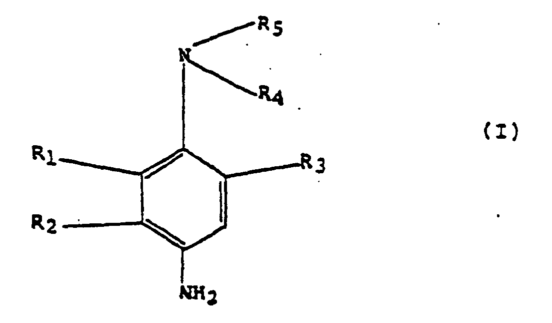 worin R₁, R₂ und R₃, gleich oder verschieden, ein Wasserstoff- oder Halogenatom, einen Alkylrest mit 1 bis 4 Kohlenstoffatomen, einen Alkoxyrest mit 1 bis 4 Kohlenstoffatomen, R₄ und R₅, gleich oder verschieden, ein Wasserstoffatom, einen Alkyl-, Hydroxyalkyl-, Alkoxyal-kyl-, Carbamylalkyl-, Mesylaminoalkyl-, Acetylaminoalkyl-, Ureidoalkyl-, Carbalkoxyaminoalkyl-, Piperidinoalkyl- oder Morpholinoalkylrest darstellen, wobei diese Alkyl- oder Alkoxygruppen 1 bis 4 Kohlenstoffatome aufweisen, oder R₄ und R₅ zusammen mit dem Stickstoffatom, an das sie gebunden sind, auch einen Piperidino- oder Morpholinoheterozyklus bilden, mit der Maßgabe, dass R₁ oder R₃ ein Wasserstoffatom darstellen, wenn R₄ und R₅ kein Wasserstoffatom darstellen, sowie die Salze dieser Verbindungen, unter Ausschluss von 2,6- und 2,3-Dimethyl-p-phenylendiamin.

4. Verfahren gemäß Anspruch 2 oder 3, **dadurch gekennzeichnet, dass** die Verbindungen der Formel I) ausgewählt sind aus Isopropyl-p-phenylendiamin, p-Phenylendiamin, 2-Methyl-p-phenylendiamin, Methoxy-p-phenylendiamin, Chlor-pphenylendiamin, 2-Methyl-5-methoxy-p-phenylendiamin, 2,6-Dimethyl-5-methoxy-p-phenylendiamin, N,N-Dimethyl-pphenylendiamin, 3-Methyl-4-amino-N,N-diethyanilin, N,N-Di(β-hydroxyethyl)-p-phenylendiamin, 3-Methyl-4-amino-N, N-di(β-hydroxyethyl)anilin, 3-Chlor-4-amino-N,N-di(β-hydroxyethyl)anilin, 4-Amino-N,N-(ethyl,carbamylmethyl)anilin, 3-Methyl-4-amino-N,N-(ethylcarbamylmethyl)anilin, 4-Amino-N,N-(ethyl,β-piperidinoethyl)anilin, 3-Methyl-4-amino-N,N-(ethyl-β -morpholinoethyl)anilin, 4-Amino-N,N-(ethyl-β-morpholinoethyl)anilin, 3-Methyl-4-amino-N,N-(ethyl-β-morpholinoethyl)anilin, 4-Amino-N,N-(ethyl,betacetylaminoethyl)anilin, 4-Amino-N-(β-methoxyethyl)anilin, 3-Methyl-4-amino-N,N-(ethyl, η-acetylaminoethyl)anilin, 4-Amino-N,N-(ethyl, β-mesylaminoethyl)anilin, 3-Methyl-4-amino-N,N-(ethyl, β-mesylaminoethyl)anilin, 4-Amino-N,N-(ethyl, β-sulfoethyl)aniin, 3-Methyl-4-amino-N,N-(ethyl, β-sulfoethyl)anilin, N-(4'-Amino)phenyl)morpholin, N-((4'-Amino)phenyl)piperidin, in Form der freien Base oder von Salzen.

5. Verfahren gemäß Anspruch 2 oder 3, **dadurch gekennzeichnet, dass** die p-Aminophenole ausgewhält sind aus p-Aminophenol, 2-Methyl-4-aminophenol, 3-Methyl-4-aminophenol, 2-Chlor-4-aminophenol, 3-Chlor-4-aminophenol, 2,6-Dimethyl-4-aminophenol, 3,5-Dimethyl-4-aminophenol, 2,3-Dimethyl-4-aminophenol, 2-Hydroxymethyl-4-aminophenol, 2-(β-Hydroxyethyl)-4-aminopenol, 2-Methoxy-4-aminophenol, 3-Methoxy-4-aminophenol, 2,5-Dimethyl-4-aminophenol, 2-Methoxymethyl-4-aminophenol.

6. Verfahren gemäß Anspruch 1, **dadurch gekennzeichnet, dass** die Oxidationsfarbstoffvorstufen Oxidationsfarbstoffvorstufen vom o-Typ sind, ausgewählt aus o-Aminophenolen und o-Phenylendiaminen.

7. Verfahren gemäß einem der Ansprüche 1 bis 6, **dadurch gekennzeichnet, dass** der pH-Wert der auf die keratinischen Fasern aufgetragenen Zusammensetzung 3 bis 6,9 beträgt.

8. Verfahren gemäß einem der Ansprüche 1 bis 7, **dadurch gekennzeichnet, dass** die zur Färbung der keratinischen Fasern verwendete Zusammensetzung zusätzlich zum 2,4-Diamino-1,3-dimethoxybenzol-Kuppler weitere Kuppler enthält, ausgewählt aus m-Diphenolen, m-Aminophenolen, m-Phenylendiaminen, m-N-Acylaminophenolen, m-Ureidophenolen, m-Carbalkoxyaminophenolen, α-Naphthol, Kupplern mit einer aktiven Methylengruppe, ausgewählt aus Diketoverbindungen und Pyrazolonen.

9. Verfahren gemäß Anspruch 8, **dadurch gekennzeichnet, dass** die Kuppler ausgewählt sind aus: 2,4-Dihydroxyphenoxyethanol, 2, 4-Dihydroxyanisol, m-Aminophenol, Resorcin, dem Monomethylether von Resorcin, 2-Mthylresorcin, Pyrocatechol, 2-Methyl-5-N-(β-hydroxyethyl)aminophenol, 2-Methyl-5-N-(β-mesylaminoethyl) aminophenol, 6-Hydroxybenzomorpholin, 2,4-Diaminoanisol, 2,4-Diaminophenoxyethanol, 6-Aminobenzomorpholin, (2-N-(β-Hydroxyethyl) amino-4-amino)phenoxyethanol, 2-Amino-4-N-(β-hydroxyethyl) aminoanisol, (2,4-Diamino)phenyl-β,γ-dihydroxypropylether, 2,4-Diaminophenoxyethylamin, 2-Methyl-5-aminophenol, 2, 6-Dimethyl-3-aminophenol, 3,4-Methylendioxyphenol und 3,4-Methylendioxyanilin sowie aus deren Salzen.

10. Verfahren gemäß einem der Ansprüche 1 bis 9, **dadurch gekennzeichnet, dass** die Zusammensetzung anionische, kationische, nicht-ionische, amphotere oberflächenaktive Mittel oder deren Mischungen, Verdickungsmittel, Antioxidantien oder jeden weiteren kosmetisch zulässigen Hilfsstoff enthält.

11. Mittel zur Färbung keratinischer Fasern und insbesondere der Haare, **dadurch gekennzeichnet, dass** es aus zwei Bestandteilen zusammengesetzt ist: einem Bestandteil (A) aus einer Zusammensetzung, die aus Wasser oder einer Mischung aus Wasser und einem Lösungsmittel, ausgewählt aus C₂₋₄-Niedrigalkoholen, Glycerin, Glycolen oder Glycolethern, Diethylenglycolmonoethylether und -monomethylether, aromatischen Alkoholen oder aus deren Mischungen, und gegebenenfalls aus anionischen, kationischen, nicht-ionischen, amphoteren oberflächenaktiven Mitteln oder deren Mischungen, Verdickungsmitteln, Antioxidantien, Eindringmitteln, Sequestriermitteln, Konservierungsstoffen und aus Parfümen zusammengesetzt ist,
- aus mindestens 2,4-Diamino-1,3-dimethoxybenzol als Kuppler und einer der in einem der Ansprüche 2 bis 6 definierten Oxidationsfarbstoff-Vorstufenverbindungen und einem Bestandteil (B) aus einem oxidierenden Mittel, das aus Wasserstoffperoxid zusammengesetzt ist, wobei der pH-Wert der Bestandteile (A) und (B) einen solchen Wert aufweist, dass nach Vermischung in Mengenanteilen von 90 bis 10% für den Bestandteil (A) und von 10 bis 90% für den Bestandteil (B) die entstandene Zusammensetzung einen pH-Wert unter 7 ergibt.

12. Mittel gemäß Anspruch 11, **dadurch gekennzeichnet, dass** der Bestandteil (A) einen pH von 3 bis 10,5 aufweist.

13. Mittel gemäß Anspruch 11 oder 12, **dadurch gekennzeichnet, dass** der Bestandteil (A) Oxidationsfarbstoff-Vorstufenverbindungen vom p- und/oder o-Typ sowie die weiteren Kuppler in Mengenverhältnissen von 0,3 bis 7 Gew.%, bezogen auf das Gesamtgewicht des Bestandteils (A), enthält.

14. Mittel gemäß einem der Ansprüche 11 bis 13, **dadurch gekennzeichnet, dass** die Konzentration an 2,4-Diamino-1,3-dimethoxybenzol 0,05 bis 3,5 Gew.%, bezogen auf das Gesamtgewicht des Bestandteils (A), beträgt.

15. Mittel gemäß einem der Ansprüche 11 bis 14, **dadurch gekennzeichnet, dass** der Bestandteil (A) oberflächenaktive Mittel in Mengenverhältnissen von 0,1 bis 55 Gew.%, Lösungsmittel zusätzlich zum Wasser in Mengenverhältnissen von 0,5 bis 40 Gew.%, Verdickungsmittel in Mengenverhältnissen von 0,1 bis 5 Gew.%, Antioxidantien in Mengenverhältnissen von 0,02 bis 1,5 Gew.% und/oder jeden weiteren kosmetisch zulässigen Hilfsstoff enthält.

16. Mittel gemäß einem der Ansprüche 11 bis 15, **dadurch gekennzeichnet, dass** der Bestandteil (B) einen pH aufweist, der einen Minimalwert von 1 bis einen Wert von weniger als 7 hat.

17. Verfahren zur Färbung keratinischer Fasern und insbesondere der Haare, **dadurch gekennzeichnet, dass** es aus einer ersten Stufe besteht, wobei man die Bestandteile des in einem der Ansprüche 11 bis 16 definierten Färbemittels in getrennter Form aufbewahrt und vor der Aufbringung die Bestandteile (A) und (B) in Mengenverhältnissen von 10 bis 90% für den Bestandteil (A) und von 90 bis 10% für den Bestandteil (B) vermischt, und zwar so, dass man eine Zusammensetzung mit einem pH unterhalb 7 erhält, und wobei man diese Mischung unmittelbar nach der Herstellung auf die keratinischen Fasern aufträgt.

18. Vorrichtung aus mehreren Teilen oder Kit zur Färbung, **dadurch gekennzeichnet, dass** sie aus 2 Teilen besteht, deren erster Teil den in einem der Ansprüche 11 bis 16 definierten Bestandteil (A) und deren zweiter Teil den in einem der Ansprüche 11 bis 17 definierten Bestandteil (B) umfassen.

19. Vorrichtung gemäß Anspruch 18, **dadurch gekennzeichnet, dass** sie mit einem Element ausgerüstet ist, das es erlaubt, die gewünschte Mischung aus den Bestandteilen (A) und (B) auf die Haare zu bringen.

20. Verfahren zur Färbung gemäß einem der Ansprüche 1 bis 10, **dadurch gekennzeichnet, dass** man auf die Haare die Zusammensetzung aufbringt und sie 3 bis 40 min verweilen lässt, die Haare spült und gegebenenfalls schamponiert, bevor erneut gespült und getrocknet wird.

21. Gebrauchsfertige Zusammensetzung zur Färbung keratinischer Fasern zur Verwendung im Verfahren gemäß einem der Ansprüche 1 bis 10.

22. Zusammensetzung gemäß Anspruch 21, enthaltend 2,4-Diamino-1,3-dimethoxybenzol in Mengenverhältnissen von 0,01 bis 3,5 Gew.%, bezogen auf das Gesamtgewicht der Zusammensetzung.

## Claims

1. Process for dyeing keratinous fibres, especially human keratinous fibres such as the hair, **characterised in that** a composition is applied to these fibres, consisting of water or a mixture of water and a solvent chosen from C₂-C₄ lower alkanols, glycerol, glycols or glycol ethers, diethylene glycol monomethyl ether and monoethyl ether, aromatic alcohols and mixtures thereof, and optionally anionic, cationic, nonionic or amphoteric surfactants or mixtures thereof, thickeners, antioxidants, penetrating agents, sequestering agents, preserving agents and fragrances,
- at least, as a coupler, 2,4-diamino-1,3-dimethoxybenzene, or one of its salts;
- one or several oxidation dye precursors;
- an oxidising agent consisting of hydrogen peroxide;
- the pH of the composition applied to the fibres being less than 7.

2. Process according to Claim 1, **characterised in that** the oxidation dye precursors are selected from para-phenylenediamines, para-aminophenols and heterocyclic para precursors.

3. Process according to Claim 2, **characterised in that** the para-phenylenediamines are selected from the compounds corresponding to the formula 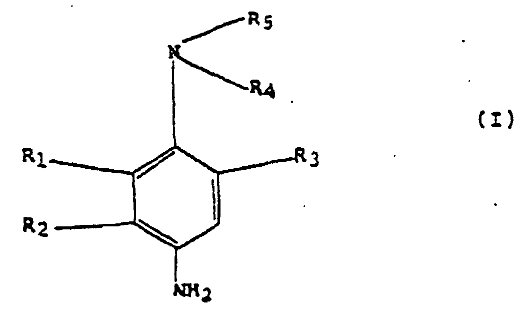 in which R₁, R₂ and R₃, which may be identical or different, represent a hydrogen or halogen atom, an alkyl radical having from 1 to 4 carbon atoms or an alkoxy radical having from 1 to 4 carbon atoms, and R₄ and R₅, which may be identical or different, represent a hydrogen atom or an alkyl, hydroxyalkyl, alkoxyalkyl, carbamylalkyl, mesylaminoalkyl, acetylaminoalkyl, ureidoalkyl, carbalkoxyaminoalkyl, piperidinoalkyl or morpholinoalkyl radical, these alkyl or alkoxy groups having from 1 to 4 carbon atoms, or alternatively R₄ and R₅, together with the nitrogen atom to which they are linked, form a piperidino or morpholino heterocycle, with the proviso that R₁ or R₃ represents a hydrogen atom when R₄ and R₅ do not represent a hydrogen atom, as well as the salts of these compounds, with the exception of 2,6-dimethyl-para-phenylenediamine and 2,3-dimethyl-para-phenylenediamine.

4. Process according to Claim 2 or 3, **characterised in that** the compounds of formula (I) are selected from isopropyl-p-phenylenediamine, p-phenylenediamine, 2-methyl-p-phenylenediamine, methoxy-para-phenylenediamine, chloro-para-phenylenediamine, 2-methyl-5-methoxy-para-phenylenediamine, 2, 6-dimethyl-5-methoxy-para-phenylenediamine, N,N-dimethyl-para-phenylenediamine, 3-methyl-4-amino-N,N-diethylaniline, N,N-bis(β-hydroxyethyl)para-phenylenediamine, 3-methyl-4-amino-N,N-bis(β-hydroxyethyl)-aniline, 3-chloro-4-amino-N,N-bis(β-hydroxyethyl)-aniline, 4-amino-N-ethyl-N-(carbamylmethyl)aniline, 3-methyl-4-amino-N-ethyl-N-(carbamylmethyl)aniline, 4-amino-N-ethyl-N-(β-piperidinoethyl)aniline, 3-methyl-4-amino-N-ethyl-N-(β-piperidinoethyl)aniline, 4-amino-N-ethyl-N-(β-morpholinoethyl)aniline, 3-methyl-4-amino-N-ethyl-N-(β-morpholinoethyl)aniline, 4-amino-N-ethyl-N-(β-acetylaminoethyl)aniline, 4-amino-N-(β-methoxyethyl)aniline, 3-methyl-4-amino-N-ethyl-N-(β-acetylaminoethyl)aniline, 4-amino-N-ethyl-N-(β-mesylaminoethyl)aniline, 3-methyl-4-amino-N-ethyl-N-(β-mesylaminoethyl)aniline, 4-amino-N-ethyl-N-(β-sulphoethyl)aniline, 3-methyl-4-amino-N-ethyl-N-(β-sulphoethyl)aniline, N-(4'-amino)phenylmorpholine and N-(4'-amino)phenylpiperidine, in the form of a free base or of salts.

5. Process according to Claim 2 or 3, **characterised in that** the p-aminophenols are selected from p-aminophenol, 2-methyl-4-aminophenol, 3-methyl-4-aminophenol, 2-chloro-4-aminophenol, 3-chloro-4-aminophenol, 2,6-dimethyl-4-aminophenol, 3,5-dimethyl-4-aminophenol, 2,3-dimethyl-4-aminophenol, 2-hydroxymethyl-4-aminophenol, 2-(β-hydroxyethyl)-4-aminophenol, 2-methoxy-4-aminophenol, 3-methoxy-4-aminophenol, 2,5-dimethyl-4-aminophenol and 2-methoxymethyl-4-aminophenol.

6. Process according to Claim 1, **characterised in that** the oxidation dye precursors are ortho type oxidation dye precursors selected from orthoaminophenols and ortho-phenylenediamines.

7. Process according to any one of Claims 1 to 6, **characterised in that** the pH of the composition applied to the keratinous fibres is between 3 and 6.9.

8. Process according to any one of Claims 1 to 7, **characterised in that** the composition used for dyeing the keratinous fibres contains, in addition to the coupler 2, 4-diamino-1,3-dimethoxybenzene, other couplers selected from meta-diphenols, meta-aminophenols, meta-phenylenediamines, meta-(N-acylamino)phenols, meta-ureidophenols, meta-carbalkoxyaminophenols, α-naphthol and couplers possessing an active methylene group, selected from diketo compounds and pyrazolones.

9. Process according to Claim 8, **characterised in that** the couplers are selected from 2,4-dihydroxyphenoxyethanol, 2,4-dihydroxyanisole, meta-aminophenol, resorcinol, resorcinol monomethyl ether, 2-methylresorcinol, pyrocatechol, 2-methyl-5-[N-(β--hydroxyethyl)amino]phenol, 2-methyl-5-[N-(β-mesylaminoethyl)amino]phenol, 6-hydroxybenzomorpholine, 2,4-diaminoanisole, 2,4-diaminophenoxyethanol, 6-aminobenzomorpholine, 2-[N-(β-hydroxyethyl)amino]-4-aminophenoxyethanol, 2-amino-4-[N-(β-hydroxyethyl)amino]-anisole, 2,4-diaminophenyl β,α-dihydroxypropyl ether, 2, 4-diaminophenoxyethylamine, 2-methyl-5-aminophenol, 2, 6-dimethyl-3-aminophenol, 3,4-methylenedioxyphenol, 3,4-methylenedioxyaniline and their salts.

10. Process according to any one of Claims 1 to 9, **characterised in that** the composition contains anionic, cationic, nonionic or amphoteric surfactants or mixtures thereof, thickening agents, antioxidants and/or any other cosmetically acceptable adjuvant.

11. Agent for dyeing keratinous fibres, and especially the hair, **characterised in that** it consists of two components: a component (A) consisting of a composition consisting of water or a mixture of water and a solvent chosen from C₂-C₄ lower alkanols, glycerol, glycols or glycol ethers, diethylene glycol monomethyl ether and monoethyl ether, aromatic alcohols and mixtures thereof, and optionally anionic, cationic, nonionic or amphoteric surfactants or mixtures thereof, thickeners, antioxidants, penetrating agents, sequestering agents, preserving agents and fragrances, - at least, as a coupler, the 2,4-diamino-1,3-dimethoxybenzene and an oxidation dye precursor as defined in any one of Claims 2 to 6, and a component (B) consisting of an oxidising agent consisting of hydrogen peroxide, the pH of the components (A) and (B) being such that, after mixing in proportions of 90 to 10% for the component (A) and 10 to 90% for the component (B), the resulting composition has a pH of less than 7.

12. Agent according to Claim 11, **characterised in that** the component (A) has a pH of between 3 and 10.5.

13. Agent according to Claim 11 or 12, **characterised in that** the component (A) contains the oxidation dye precursors of the para and/or ortho type as well as the couplers, in proportions of between 0.3 and 7% by weight relative to the total weight of the component (A).

14. Agent according to any one of Claims 11 to 13, **characterised in that** the concentration of 2,4-diamino-1,3-dimethoxybenzene is between 0.05 and 3.5% by weight relative to the total weight of the component (A).

15. Agent according to any one of Claims 11 to 14, **characterised in that** the component (A) contains surfactants in proportions of 0.1 to 55% by weight, solvents in addition to water in proportions of between 0.5 and 40% by weight, thickening agents in proportions of between 0.1 and 5% by weight, antioxidants in proportions of between 0.02 and 1.5% by weight and/or any other cosmetically acceptable adjuvant.

16. Agent according to any one of Claims 11 to 15, **characterised in that** the component (B) has a pH which has a minimum value of 1 and less than 7.

17. Process for dyeing keratinous fibres, and especially the hair, **characterised in that** it consists in a first step consisting in storing separately the components of the dyeing agent as defined in any one of Claims 11 to 16, and in mixing the components (A) and (B) in proportions of 10 to 90% for the component (A) and 90 to 10% for the component (B) before application, so as to obtain a composition having a pH of less than 7, and applying this mixture immediately after preparation to the keratinous fibres.

18. Multi-compartment device or dyeing kit, **characterised in that** it consists in two compartments, of which a first compartment contains the component (A) as defined in any one of Claims 11 to 16, and the second compartment contains the component (B) as defined in Claims 11 and 17.

19. Device according to Claim 18, **characterised in that** it is equipped with a means enabling the desired mixture of the components (A) and (B) to be delivered on the hair.

20. Dyeing process according to any one of Claims 1 to 10, **characterised in that** the composition is applied to the hair and it that it is left in place for 3 to 40 minutes, **in that** the hair is rinsed and **in that** it is optionally shampooed before a further rinse and drying.

21. Ready-to-use composition for dyeing keratinous fibres as employed in the process according to any one of Claims 1 to 10.

22. Composition according to Claim 21, containing 2,4-diamino-1,3-dimethoxybenzene in proportions of 0.01 to 3.5% by weight relative to the total weight of the composition.
